(19) Europäisches Patentamt
European Patent Office
Office européen des brevets

(11) Publication number: 0 113 397
B1

(12) # EUROPEAN PATENT SPECIFICATION

(45) Date of publication of patent specification: 19.04.89

(51) Int. Cl.$^4$: A 61 K 9/06, A 61 K 9/08, A 61 K 47/00

(21) Application number: 83110250.4

(22) Date of filing: 14.10.83

(54) **Anthelmintic gel compositions and a method for their preparation at ambient temperatures.**

(30) Priority: 13.12.82 US 449067

(43) Date of publication of application:
18.07.84 Bulletin 84/29

(45) Publication of the grant of the patent:
19.04.89 Bulletin 89/16

(84) Designated Contracting States:
AT BE CH DE FR GB IT LI NL SE

(73) Proprietor: AMERICAN CYANAMID COMPANY
1937 West Main Street P.O. Box 60
Stamford Connecticut 06904-0060 (US)

(72) Inventor: Quinlan, James Michael
135 Edinburg Road
Trenton New Jersey 08619 (US)

(74) Representative: Wächtershäuser, Günter, Dr.
Tal 29
D-8000 München 2 (DE)

(56) References cited:
EP-A-0 061 806
US-A-3 639 574
US-A-3 639 575
US-A-3 740 421
US-A-3 836 637
US-A-3 867 533
US-A-3 980 791
US-A-4 287 176

SEIFEN ÖLE FETTE WACHSE, vol. 106, no. 8, May 14, 1980, pp. 225-226

(56) References cited:
J. JANISTYN "Handbuch der Kosmetika und Riechstoffe", ed. 3, vol. 1, 1978, Hüthig, Heidelberg, pp. 725-729

EP 0 113 397 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a method for the preparation of clear aqueous homogeneous gels characterized by subzero gelation points.

Aqueous gel compositions containing an anthelmeintic compound of formula (I)

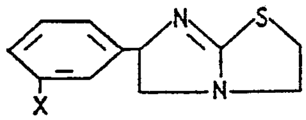

wherein X is hydrogen —NHR; R is $C_2$—$C_5$ alkanoyl or benzoyl, and pharmaceutically acceptable salts thereof, together with a gellant, water and propylene glycol as well as the preparation of said gel is disclosed in US—A—4,287,176.

Among the compounds of formula (I) preferred are levamisole, tetramisole, butamisole or benzamisole.

The use of gels characterized by sub-zero gelation, as anthelmintic preparations is disclosed in US—A—4,287,176 (1981). Specifically this patent discloses the use of anthelmintic gels and their formation at −23 to −20°C. The gellant used in these formulations is a nonionic surfactant of structure: α-hydro-Ω-hydroxy-poly(oxyethylene)poly(oxypropylene)poly(oxyethylene)block copolymer, average molecular weight 12,500; specific gravity 1.05; mp 56°C; Brookfield viscosity (3100) 3.1 Pa.s at 77°C; surface tension of a 0.1% aqueous solution: $40{,}6 \cdot 10^{-4}$ N/m (40.6 dynes/cm) at 25°C (as measured on a du Nouy tensiometer; US—A—3,740,421 and others).

Recommended processes for preparing gels using this gellant are either a "cold" or "hot" technique in a temperature range where the gel is in a liquified state prior to gelation as described below and see Irving R. Schmolka, J. Biomed. MATER. RES., Vol. 6, pgs. 571—582 (1972).

The "cold" technique, is recommended as a first step to determine the feasibility of the potential gel system and to establish the optimum gellant concentration required. In this technique, the gellant is dissolved, along with the other ingredients, in water at a temperature of 5—10°C. Aeration should be avoided. When solution is completed, usually after about two hours of stirring at 10°C, the system is allowed to warm up to room temperature, whereupon it forms a ringing gel. Water-insoluble ingredients may be dissolved in alcohol or acetone, cooled and added to the gellant solution. However, an alcohol content of 20% or greater should be avoided since these weaken the gel. Where alcohol cannot be tolerated, the water-insoluble materials should be heated with the gellant and cold water slowly added to the molten mixture to bring the temperature below that at which gelation occurs. This procedure has been used successfully with many water-insoluble organic compounds, including N,N-diethyltoluamide, amyl para-dimethylamino-benzoate and lauryl lactate.

The "hot" technique readily lends itself to production. In the laboratory, all ingredients are placed into a three-neck glass flask, equipped with a mechanical stirrer, reflux condenser and dropping funnel to prevent cpmposition change due to loss of water. While dissolving the gellant, the mixture is heated to about 80°C, and stirred gently. Excessive agitation will cause the development of foam and should be avoided. When the system is homogeneous, transfer to containers. Upon cooling to room temperature, the product gels.

It has been proposed in SÖFW, vol. 106, No. 8, 1980, pages 225 to 226, to increase the amount of gellant when a more acidic or a more rigid gel is required.

The present invention provides a method for the preparation, at ambient temperatures, of clear aqueous homogeneous gels characterized by subzero gelation points, comprising, mixing 15—50% by weight of the gellant α-hydro-Ω-hydroxy-poly(oxyethylene)poly(oxypropylene)poly(oxyethylene) block copolymer, average molecular weight 12,500; mp 56°C, Brookfield viscosity of 3.1 Pa.s (3100) at 77°C; surface tension of a 0.1% aqueous solution: $40{,}6 \cdot 10^{-4}$ N/m (40.6 dynes/cm) (measured with a du Nouy tensiometer) with 14—31% by weight of propylene, up to 41% by weight of one or more active ingredients, and admixing the thus formed mixture with 30—50% by weight of water, characterized by stirring the resulting mixture at ambient temperatures (20—60°C) without additional heating or cooling, until it is homogeneous at atmospheric pressure or under reduced pressure of from 13.3—133 mbar (10—100 mm Hg).

Unexpectedly we find that gels suitable but not limited to anthelmintic use may be prepared at ambient temperatures (20—60°C) under reduced pressure as demonstrated by the general procedure described below.

A gellant phase may be prepared by dissolving the gellant 15—50% and preferably 20—30% by weight of final formulation in propylene glycol 14—31% by weight at 60—80°C or alternatively, the gellant phase prepared completely at ambient temperatures as described below.

The gellant phase is prepared by slurrying the gellant 15—50% and preferably 15—35% by weight of formulation in propylene glycol 14—31% by weight for 15 minutes to one hour under reduced pressure 33.25—66.5 mbar (25—50 mm Hg) at room temperature. The gellant selected is a nonionic surfactant of

structure α-hydro-Ω-hydroxy-poly(oxyethylene)poly(oxypropylene)poly(oxyethylene) block copolymer, average molecular weight 12,500; mp 56°C; Brookfield viscosity of (3100) 3.1 Pa.s at 77°C; surface tension of a 0.1% aqueous solution: 40.6 dynes/cm (measured with a du Nouy tensiometer).

An aqueous solution containing the remaining ingredients may be prepared by dissolving a levamisole or tetramisole salt, preferably the hydrochloride, in amounts of from about 3% by weight to about 25% by weight and preferably 6—12% by weight of final formulation in deionized or distilled water used in amounts of from about 15% by weight to about 50% by weight and preferably 35—45% by weight of formulation. This solution is buffered by dissolving 1.5% by weight of citric acid and 1.0% by weight of trisodium citrate to provide a pH range at which long term chemical stability of the components is achieved, i.e. pH 3—3.5.

Optional components, which may be incorporated into the above solution at this stage are:

a. Benzyl alcohol added in amounts of from about 0.5% by weight to about 1.5% by weight and preferably 1.5% by weight of formulation, as an antimicrobial preservative;

b. the yellow dye C.I. Acid yellow No. 23; ("tartrazine") F.D. & C yellow No. 5; 4,5-dihydro-5-oxo-1-(4-sulfophenyl)-4-[(sulfophenyl)azo]-1H-pyrazole-3-carboxylic acid trisodium salt) used as coloring agent in amounts of from about 0.01% by weight to about 0.03% by weight and preferably 0.01% by weight of formulation;

c. an antifoaming agent comprising a mixture of dimethylpolysiloxanes of structure:

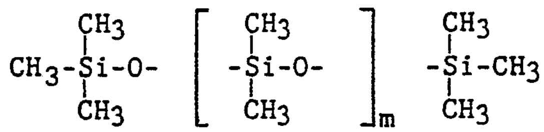

and silica gel, wherein the calculated average value of m is 200—350, the mixture is a water-white viscous oil-like liquid; d=0.965—0.970; $n_D^{25}$ about 1.404; viscosity about 60 Pa.s (60,000 centistokes) (and said antifoaming agent is described in United States patent 2,441,098) used in amounts of from 0.001—0.02% by weight and preferably 0.02% by weight of formulation.

The anthelmintic gel is prepared by simply mixing either of the above gellant phases and the aqueous solution from one-half to two hours under reduced pressure of from 13.3—133 mbar (10—100 mm Hg) and preferably 33.25—66.5 mbar (25—50 mm Hg) at ambient temperatures of from 20—60°C, without the requirements of either additional heating or cooling. This procedure gives an air free gel which is suitable for administering exact dosages of anthelmintic by volume. When careful control of dosage of active ingredients to be administered by volume is not necessary and when the presence of air in the gel is acceptable in the final formulation, the preparation may be carried out at pressures up to and including atmospheric pressure.

By the above method a typical gel of the invention may be prepared by dissolving 11.6 g of levamisole or tetramisole hydrochloride, 1.5 g citric acid monohydrate, 1.0 g sodium citrate dihydrate, 1.5 g of benzyl alcohol and 0.01 g of the yellow dye C.I. Acid yellow No. 23 in 39 g of water. Next, a solution of the above gellant 26 g in propyleneglycol 19.39 g is prepared by mixing at 60°C. Then the solutions are mixed together under a pressure of 33.25—66.5 mbar (25—50 mm Hg) until a homogeneous mixture is obtained at 20—60°C without additional heating or cooling. The gel formed has a gelation temperature range of from −15 to −18°C; visosity of the gel is $(0.51 \times 10^{+6})$ $0.51 \cdot 10^3$ Pa.s and the water gellant ratio is 1.5/1.0.

The above procedure is equally suitable to prepare gels of the inventions containing but not limited to other anthelmintic compounds defined and described by formula (I) above.

This procedure for preparing these aqueous gels offers several advantages over existing methods. Cold techniques often limit the concentration range of ingredients due to increasing viscosities and decreases in solubility of the components as the temperature of gelation is lowered; this would be particularly true when operating temperatures approaching the freezing point of the mixture are required. By the ambient temperature method of the present invention, gels with sub-zero gelation points containing 20 to 30% gellant with water to gellant ratios of 0.54/1 to 1.4/1 may be prepared. This is a distinct advantage over prior methods of preparing gels with sub-zero gelation points since clear homogenous gels with water to gellant ratios as low as 1/4/1 containing 20 to 30% by weight of the gellant are difficult to prepare, frequently requiring more than 36 hours at temperatures below the gelation point to obtain complete solution. Thus, the method of the present invention provides for the preparation of gel compositions with sub-zero gelation points which are not obtainable by prior methods available in the art. Gel compositions with sub-zero gelation points containing 15 to 50% by weight of the gellant and water to gellant ratios of 0.54/1 to 1.4/1 are now readily obtainable. Additionally, processing at temperatures below ambient often require the use of specialized cooling systems which are more expensive and difficult to maintain and operate.

Chemical thermal instability of one or more of the components often limit the use of a hot technique particularly since gellants of this type are less soluble at elevated temperatures, adding to processing times at higher temperatures.

An additional advantage of these gels and their method of preparation at ambient temperatures

(20—60°C) is that they may also be fortified if necessary at ambient temperatures. Frequently in the manufacture of products it is necessary to adjust the concentration of the components to be within finite limits; this would be particularly true in food related and pharmaceutical products. The aqueous gels of this invention may conveniently be fortified by simple addition of the required ingredient, either as a solid or as an aqueous solution, followed by mixing under reduced pressure until a homogeneous mixture is obtained without being liquified.

The above stated advantageous features of said aqueous gels make them eminently suitable for use as carriers for active ingredients which is meant to include the biologically active component and desired additives including coloring agents, flavoring agents, conditioners, etc., as indicated by the following nonlimiting limiting examples.

Cosmetics, hair preparations, dental products, veterinary products such as anthelmintics, pharmaceutical products administered as gels, pesticide products such as insecticides, herbicides, etc., could conveniently be incorporated and applied in said aqueous gels. Further, the method of preparation of these gels readily lends itself to any method of production, batch, semi-batch or continuous processing, which enhances their attractiveness for use in the above areas.

Example 1
Preparation of levamisole gels

General Procedure

The appropriate amounts of levamisole salt as the hydrochloride, citric acid monohydrate, trisodium citrate, benzyl alcohol, yellow dye (C.I. Acid yellow No. 23) are dissolved in deionized or distilled water and the solution clarified if necessary. Next, a homogeneous gellant solution containing the appropriate amount of gellant and propylene glycol is prepared by mixing at 60-80°C. The gellent selected is a nonionic surfactant of structure: α-hydro-Ω-hydroxy-poly(oxyethylene)poly(oxypropylene)poly(oxyethylene) block copolymer, average molecular weight 12,500; mp 56°C; Brookfield viscosity (3100) 3.1 Pa.s at 77°C; surface tension of a 0.1% aqueous solution: $40.6 \cdot 10^{-4}$ N/m (40.6 dynes/cm) (measured with a du Nouy tensiometer). Then the two solutions are mixed together at ambient temperatures (20—60°C) under reduced pressure of 33.25—66.5 mbar (25—50 mm Hg) until a homogeneous mixture is obtained.

The composition of the formulations and other data obtained are summarized in Table I below.

TABLE I

Composition and physical parameters of various levamisole gels

| Component | Percent by weight Composition of formulations | | | | | | | | | | |
|---|---|---|---|---|---|---|---|---|---|---|---|
| | No. 1 | No. 2 | No. 3 | No. 4 | No. 5 | No. 6 | No. 7 | No. 8 | No. 9 | No. 10 | No. 11 |
| Levanisole HCl | 10.49 | 12.00 | 11.60 | 11.60 | 11.60 | 11.73 | 11.71 | 11.71 | 11.73 | 11.73 | 23.46 |
| Water | 50.00 | 45.00 | 45.00 | 40.00 | 39.00 | 37.50 | 28.26 | 18.86 | 34.50 | 40.31 | 30.50 |
| Gellant | 20.00 | 20.00 | 25.00 | 25.00 | 26.00 | 25.00 | 30.00 | 35.00 | 28.00 | 29.00 | 28.00 |
| Citric Acid Monohydrate | 1.50 | 1.50 | 1.50 | 1.50 | 1.50 | 1.50 | 1.50 | 1.50 | 1.50 | 1.50 | 1.50 |
| Trisodium citrate, dihydrate | 1.00 | 1.00 | 1.00 | 1.00 | 1.00 | 1.00 | 1.00 | 1.00 | 1.00 | 1.00 | 1.00 |
| Benzyl alcohol | — | — | 1.50 | 1.50 | 1.50 | 1.50 | 1.50 | 1.50 | 1.50 | 1.50 | 1.50 |
| C.I. Acid yellow No. 23 | 0.01 | 0.01 | 0.01 | 0.01 | 0.01 | 0.01 | 0.01 | 0.01 | 0.01 | 0.01 | 0.01 |
| Antifoaming Agent | — | — | — | — | — | 0.02 | 0.02 | 0.02 | 0.02 | 0.02 | 0.02 |
| Propylene glycol | 17.00 | 20.40 | 14.39 | 19.39 | 19.39 | 21.74 | 26.00 | 30.40 | 21.74 | 14.93 | 14.01 |
| Total | 100.00 | 100.00 | 100.00 | 100.00 | 100.00 | 100.00 | 100.00 | 100.00 | 100.00 | 100.00 | 100.00 |
| Water gellant ratio | 2.5/1.0 | 2.25/1.0 | 1.8/1.0 | 1.6/1.0 | 1.5/1.0 | 1.5/1.0 | 0.94/1 | 0.54/1 | 1.23/1 | 1.39/1 | 1.09/1 |
| Gelation temperature °C | +11 to +13 | +6 to +10 | −7 to −9 | −11 to −14 | −15 to −18 | −15 to −18 | <−27 | <−27 | −25 | — | — |

## Example 2
## Fortification of subpotent levamisole gels

General Procedures

Following the procedure of Example 1, the appropriate amounts of levamisole hydrochloride, citric acid, trisodium citrate, benzyl alcohol and yellow dye (C.I. Acid yellow No. 23) are dissolved in deionized water. Following the same procedure, the appropriate amounts of citric acid, trisodium citrate, benzyl alcohol and yellow dye (C.I. Acid Yellow No. 23) are dissolved in deionized water. These two aqueous solutions are combined in the proper proportions to give the desired amount of solution containing 80% of the desired levamisole hydrochloride. Next, a homogeneous gellant solution containing the appropriate amounts gellant as defined in Example 1 and propylene glycol is prepared by mixing at 60—80°C.

The above gellant and aqueous solutions are then mixed together at ambient temperatures (20—60°C) under reduced pressure of 33.25—66.5 mbar (25—50 mm Hg) for one hour. A sample is withdrawn for analysis. Sample 1.

A 50.40% w/w levamisole hydrochloride aqueous solution was added to bring the levamisole hydrochloride content from 80—90% of the required amount and the mixture stirred for one hour at ambient temperatures without additional heating or cooling, under reduced pressure 33.25—66.5 mbar (25—50 mm Hg). A sample is taken for analysis. Sample 2.

Solid technical levamisole hydrochloride is then added to bring the potency to 100% of the required amount and the mixture stirred at ambient temperature without additional heating or cooling for one hour under reduced pressure 33.25—66.5 mbar (25—50 mm Hg). A sample is taken for analysis. Sample 3.

The results of the fortifications of the formulations and other data are summarized in Table II below.

TABLE II

Fortification of levamisole hydrochloride gel from 9.74 to 12.27%

| | Sample 1 | Sample 2 added as aqueous solution | Sample 3 added as solid |
|---|---|---|---|
| Theoretical % levamisole hydrochloride w/w | 9.43 | 10.72 | 12.20 |
| Assay % w/w levamisole hydrochloride | 9.74 | 10.63 | 12.127 |
| Gel point of resulting gel | −19 to −20°C | −17 to −18°C | −18 to −19°C |
| Viscosity of resulting gel (Brookfield RVT—RL—345 T.E. spindle at 0.5 rpm) | $3.24 \times 10^3$ Pa.s ($3.24 \times 10^{+6}$ cps) | $3.10 \times 10^3$ Pa.s ($3.10 \times 10^{+6}$ cps) | $3.02 \times 10^3$ Pa.s ($3.02 \times 10^{+6}$ cps) |

## Example 3
## Preparation of aqueous gels at ambient temperatures

The appropriate amounts of gellant as defined in Example 1, either as a homogeneous solution at 60—80°C, or as a slurry prepared by stirring under reduced pressure 33.25—66.5 mbar (25—50 mm Hg) for 15 minutes to one hour at room temperature, in propylene glycol is added to the desired amount of water at ambient temperature. The resulting mixture is then stirred under reduced pressure of 33.25—66.5 mbar (25—50 mm Hg) at ambient temperature 20—60°C until a homogeneous mixture is obtained. The composition and viscosity data of aqueous gels prepared by this procedure are included in Table III.

TABLE III

Clear aqueous gels

| Component | Percent by weight composition | | |
|---|---|---|---|
| Gellant (slurry or solution) | 35.00 (solution) | 35.00 (slurry) | 40.00 (slurry) |
| Propylene glycol | 30.44 | 30.44 | 30.00 |
| Water | 34.56 | 34.56 | 30.00 |
| Total | 100.00 | 100.00 | 100.00 |
| Water gellant ratio | 0.987/1 | 0.987/1 | 0.75/1 |
| Viscosity of gel (Brookfield T. E. spindle at 0.5 rpm) Model RVT | $5.6 \times 10^3$ Pa.s | $5.6 \times 10^3$ Pa.s | $7.2 \times 10^3$ Pa.s |

**Claims**

1. A method for the preparation, at ambient temperatures, of clear aqueous homogeneous gels characterized by subzero gelation points, comprising, mixing 15—50% by weight of the gellant α-hydro-Ω-hydroxy-poly(oxyethylene)poly(oxypropylene)poly(oxyethylene) block copolymer, average molecular weight 12,500; mp 56°C, Brookfield viscosity of 3.1 Pa.s (3100) at 77°C; surface tension of a 0.1% aqueous solution: $40{,}6 \cdot 10^{-4}$ N/m (40.6 dynes/cm) (measured with a du Nouy tensiometer) with 14—31% by weight of propylene glycol, up to 41% by weight of one or more active ingredients, and admixing the thus formed mixture with 30—50% by weight of water, characterized by stirring the resulting mixture at ambient temperatures (20—60°C) without additional heating or cooling, until it is homogeneous at atmospheric pressure or under reduced pressure of from 13.3—133 mbar (10—100 mm Hg).

2. A method for the preparation of an aqueous composition, comprising: dissolving 3—25% by weight of a pharmaceutically acceptable salt of the anthelmintic compound of formula:

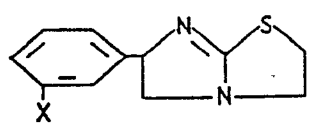

wherein X is hydrogen or —NH—R; R is $C_2$—$C_5$ alkanoyl or benzoyl; or the optical isomers thereof; 1.5% by weight of citric acid and 1.0% by weight of trisodium citrate; 0.5% by weight of benzyl alcohol; 0.01—0.03% by weight of C.I. Acid yellow No. 5; 0.001—0.02% by weight of an antifoaming agent comprising a mixture of dimethylpolysiloxanes of formula:

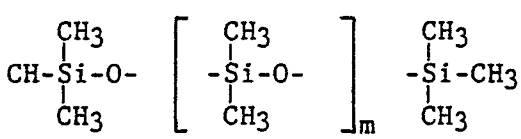

and silica gel wherein the calculated average value of m is 200—350, the mixture is a viscous liquid, d=0.965—0.970, $n_D^{25}$ about 1.404, viscosity about 60 Pa.s (60,000 centistokes), in 15—50% by weight of water and preferably 20—30% by weight of the gellant α-hydro-Ω-hydroxy-poly(oxyethylene)poly(oxypropylene)poly(oxyethylene) block copolymer, average molecular weight 12,500; specific gravity 1.05; mp 56°C; viscosity 3.1 Pa.s (3100) at 77°C; dissolved or slurried in 14—31% by weight of propylene glycol at from 60—80°C; characterized by stirring said mixture at ambient temperatures of from 20—60°C, under reduced pressure of from 33.25—66.5 mbar (25—50 mm Hg) until a clear solution occurs; with the provisos that the water/gellant ratio is from 0.94/1.0 to 2.5/1 and that the components of said composition add up to a total of 100%.

3. An aqueous composition comprising: 3—25% by weight of a pharmaceutically acceptable salt of the anthelmintic compound of the formula:

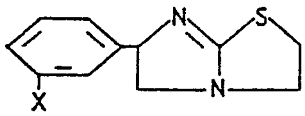

wherein X is hydrogen or —NH—R; R is $C_2$—$C_5$ alkanoyl or benzoyl; or the optical isomers thereof; 30—50% by weight of water; 14—31% by weight of propylene glycol and 15—30% by weight of the gellant α-hydro-Ω-hydroxypoly(oxyethylene)poly(oxypropylene)poly(oxyethylene) block copolymer, average molecular weight 12,500; specific gravity 1.05; mp 56°C, viscosity of 3.1 Pa.s (3100) at 77°C; wherein the compositions are gels in the temperature range of from −20 to +60°C; with the proviso that the water/gellant ratio is from 0.54/1.0 to 1.39/1.0; and that the components of the composition add up to a total of 100% by weight preparable by the process of claim 1.

**Patentansprüche**

1. Verfahren, um bei Umgebungstemperaturen klare, wässrige, homogene Gele herzustellen, die durch Unter-Null-Gelierpunkte gekennzeichnet sind, umfassend das Vermischen von 15 bis 50 Gew.-% des Geliermittels α-Hydro-Ω-hydroxypoly(oxyethylen)poly(oxypropylen)poly(oxyethylen)-Blockcopolymerisat, durchschnittliches Molekulargewicht 12500; Festpunkt 56°C, Brookfield-Viskosität 3,1 Pa.s (3100) bei 77°C; Oberflächenspannung einer 0,1 %igen wässrigen Lösung: 40,6 × $10^{-4}$ N/m (40,6 dyn/cm) (gemessen mit einem du Nouy-Tensiometer); mit 14 bis 31 Gew.-% Propylenglykol, bis zu 41 Gew.-% eines oder mehrerer Wirkstoffe und Vermischen der so gebildeten Mischung mit 30 bis 50 Gew.-% Wasser, dadurch gekennzeichnet, daß man das resultierende Gemisch bei Umgebungstemperaturen (20 bis 60°C) ohne zusätzliches Heizen oder Abkühlen rührt, bis es homogen ist, und zwar bei atmosphärischem Druck oder unter reduziertem Druck von 13,3 bis 133 mbar (10 bis 100 mm Hg).

2. Verfahren zur Herstellung eines wässrigen Mittels, umfassend das Auflösen von 3 bis 25 Gew.-% eines pharmazeutisch akzeptablen Salzes der anthelmintischen Verbindung der Formel

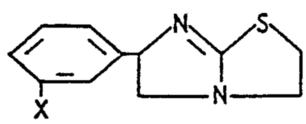

wobei X für Wasserstoff oder —NH—R steht; R für $C_2$—$C_5$-Alkanoyl oder Benzoyl steht; oder der optischen Isomeren derselben; 1,5 Gew.-% Zitronensäure und 1,0 Gew.-% Trinatriumcitrat; 0,5 Gew.-% Benzylalkohol; 0.01 bis 0,03 Gew.-% C.I. Acid yellow Nr. 5; 0,001 bis 0,02 Gew.-% eines Antischäummittels, umfassend eine Mischung von Dimethylpolysiloxanen der Formel:

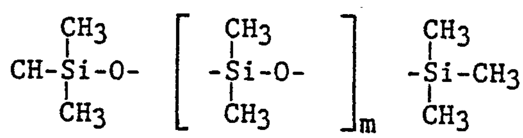

und Silicagel, wobei der berechnete Durchschnittswert von m 200 bis 350 beträgt und wobei das Gemisch eine viskose Flüssigkeit ist, d = 0,965 bis 0,970, $n_D25$ etwa, 1,404, Viskosität etwa 60 Pa.s (60.000 Centistokes), in 15 bis 50 Gew.-% Wasser und vorzugsweise 20 bis 30 Gew.-% des Geliermittels α-Hydro-Ω-hydroxy-poly(oxyethylen)poly(oxypropylen)poly(oxyethylen)-Blockcopolymerisat, durchschnittliches Molekulargewicht 12500; spezifisches Gewicht 1,05; Festpunkt 56°C; Viskosität 3,1 Pa.s (3100) bei 77°C; aufgelöst oder aufgeschlämmt in 14 bis 31 Gew.-% Propylenglykol bei 60 bis 80°C; dadurch gekennzeichnet, daß man das Gemisch bei Umgebungstemperaturen von 20 bis 60°C unter reduziertem Druck von 33,25 bis 66,5 mbar (25 bis 50 mm Hg) rührt, bis eine klare Lösung auftritt; mit den Maßgaben, daß das Wasser/Geliermittel-Verhältnis von 0,94/1,0 bis 2,5/1 beträgt und die Komponenten des Mittels sich zu einer Gesamtmenge von 100% addieren.

3. Wässriges Mittel, umfassend 3 bis 25 Gew.-% eines pharmazeutisch akzeptablen Salzes der anthelmintischen Verbindung der Formel:

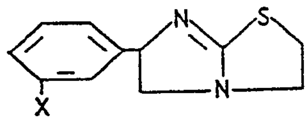

wobei X für Wasserstoff oder —NH—R steht; R für $C_2$—$C_5$-Alkanoyl oder Benzoyl steht; oder der optischen Isomeren derselben; 30 bis 50 Gew.-% Wasser; 14 bis 31 Gew.-% Propylenglykol und 15 bis 30 Gew.-% des Geliermittels α-Hydro-Ω-hydroxy-poly(oxyethylen)poly(oxypropylen)poly(oxyethylen)-Blockcopolymerisat, durchschnittliches Molekulargewicht 12500; spezifisches Gewicht 1,05; Festpunkt 56°C, Viskosität 3,1 Pa.s (3100) bei 77°C; wobei die Mittel im Temperaturbereich von −20 bis +60°C Gele sind; mit der Maßgabe, daß das Wasser/Geliermittel-Verhältnis von 0,54/1,0 bis 1,39/1,0 beträgt und daß die Komponenten des Mittels sich zu einer Gesamtmenge von 100 Gew.-% addieren, herstellbar nach dem Verfahren von Anspruch 1.

**Revendications**

1. Procédé pour la préparation, à des températures ambiantes, de gels aqueux homogènes, translucides, caractérisés par des points de gélification inférieurs à 0°C, comprenant le mélangeage de 15—50% en poids du gélifiant copolymère séquencé α-hydro-Ω-hydroxy-poly(oxyéthylène)poly(oxypropylène)poly(oxyéthylène) de masse moléculaire moyenne: 12 500; P.F.: 56°C; viscosité Brookfield: 3,1 Pa.s (3 100 centistokes) à 77°C; tension superficielle d'une solution aqueuse à 0,1%: $40{,}6 \cdot 10^{-4}$ N/m (40,6 dynes/cm) (mesurée à l'aide d'un tensiomètre de du Nouy) avec 14—31% en poids de propylèneglycol, jusqu'à 41% en poids d'un ou plusieurs composants actifs, et mélangeage du mélange ainsi obtenu avec 30—50% en poids d'eau, caractérisé par l'agitation du mélange résultant à des températures ambiantes (20—60°C) sans chauffage ni refroidissement supplémentaire, jusqu'à ce qu'il soit homogène sous la pression atmosphérique ou sous une pression réduite de 13,3 à 133 mbars (10—100 mmHg).

2. Procédé pour la préparation d'une composition aqueuse, comprenant la dissolution de 3—25% en poids d'un sel pharmaceutiquement acceptable du composé anthelminthique de formule

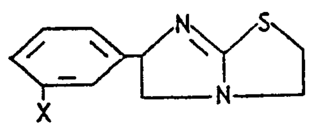

dans laquelle X est un atome d'hydrogène ou —NH—R, R étant un groupe alcanoyle en $C_2$—$C_5$ ou benzoyle, ou des isomères optiques de celui-ci; 1,5% en poids d'acide citrique et 1,0% en poids de citrate trisodique; 0,5% en poids d'alcool benzylique, 0,01—0,03% en poids de jaune acide C.I. n° 5; 0,001—0,02% en poids d'un agent antimousse comprenant un mélange de diméthylpolysiloxanes de formule:

$$CH_3-\underset{CH_3}{\overset{CH_3}{\underset{|}{\overset{|}{Si}}}}-O-\left[\begin{array}{c}CH_3\\ |\\ -Si-O-\\ |\\ CH_3\end{array}\right]_m\begin{array}{c}CH_3\\ |\\ -Si-CH_3\\ |\\ CH_3\end{array}$$

et de gel de silice, la valeur moyenne calculée de m étant 200—350, le mélange étant un liquide visqueux; d = 0,965—0,970; $n_D^{25}$ = 1,404 environ; viscosité: environ 60 Pa.s (60 000 centistokes), dans 15—50% en poids d'eau et de préférence 20—30% en poids du gélifiant copolymère séquencé α-hydro-Ω-hydroxy-poly(oxyéthylène)poly(oxypropylène)poly(oxyéthylène) de masse moléculaire moyenne: 12 500; densité: 1,05; P.F.: 56°C; viscosité: 3,1 Pa.s (3 100 centistokes) à 77°C; dissous ou en suspension dans 14—31% en poids de propylèneglycol, à une température de 60—80°C; caractérisé par l'agitation dudit mélange à des températures ambiantes (20—60°C), sous une pression réduite de 33,25 à 66,5 mbars (25—50 mm Hg), jusquà la formation d'une solution limpide; étant entendu que le rapport eau-gélifiant est de 0,94:1,0 à 2,5:1 et que les composants de ladite composition font un total de 100%.

3. Composition aqueuse comprenant 3—25% en poids d'un sel pharmaceutiquement acceptable du composé anthelminthique de formule:

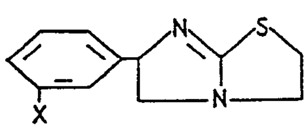

dans laquelle X est un atome d'hydrogène ou —NH—R, R étant un groupe alcanoyle en $C_2$—$C_5$ ou benzoyle,

ou des isomères optiques de ceux-ci, 30—50% en poids d'eau; 14—31% en poids de propylèneglycol et 15—30% en poids du gélifiant copolymère séquencé α-hydro-Ω-hydroxy-poly(oxyéthylène)poly(oxypropylène)poly(oxyéthylène) de masse moléculaire moyenne: 12 500; densité: 1,05; P.F.: 56°C; viscosité: 3,1 Pa.s (3 100 centistokes) à 77°C; les compositions étant des gels dans la plage de températures de −20 à +60°C, étant entendu que le rapport eau à gélifiant est de 0,54:1,0 à 1,39:1,0 et que les composants de la composition font un total de 100% en poids, préparable par le procédé de la revendication 1.